# EUROPEAN PATENT SPECIFICATION

(11) **EP 4 237 058 B1**
(45) Date of publication and mention of the grant of the patent: **15.04.2026**
(21) Application number: 21884223.5
(22) Date of filing: 28.10.2021
(51) Int. Cl.: A61M 25/00, A61F 2/86, A61F 2/90, A61F 2/91, A61F 2/915, A61M 25/01, A61M 25/06

(54) **A CATHETER ACCESSORY TO INCREASE A PUSHABILITY OF A CATHETER**
KATHETERZUBEHÖR ZUR ERHÖHUNG DER SCHIEBEFÄHIGKEIT EINES KATHETERS
ACCESSOIRE DE CATHÉTER POUR AUGMENTER LA POUSSABILITÉ D'UN CATHÉTER

(30) Priority: 28.10.2020 US 202063106431 P
(43) Date of publication of application: 06.09.2023
(73) Proprietor: Les Entreprises Nanostent Inc., Québec QC G1W 1N2 (CA)
(72) Inventor: BERTRAND, Olivier François, Quebec, Québec G1W 1N2 (CA); GALAZ, Ramses, Hermosillo, 83150 (MX)
(74) Representative: Lavoix
(86) International application number: PCT/CA2021/051523
(87) International publication number: WO 2022/087739

(56) References cited:
- US-A1- 2010 268 201
- US-A1- 2010 268 201
- US-A1- 2010 331 948
- US-A1- 2010 331 948
- US-A1- 2014 024 968
- US-A1- 2014 024 968
- US-A1- 2016 113 793
- US-A1- 2020 222 664
- US-A1- 2020 222 664

## Description

### RELATED APPLICATION

### TECHNICAL FIELD

The present disclosure relates to medical catheters and, more specifically, accessories for catheters for medical use.

### BACKGROUND

Various catheters may be used to access an inside space of blood vessels of a patient. For example, catheters may be used to access clogged heart arteries with a balloon catheter and to open the clogged heart arteries. Catheters may be also used to inspect the blood vessels. To reach a target in the blood vessel (for example, a clog), a distal portion of the catheter needs to pass from an entry point located close to patient's skin (for example, a patient's arm) via the blood vessels towards the target. The distal portion of the catheter follows the curves of the blood vessels due to manipulation of a proximal portion of the catheter by an operator. Various catheters may be used with such a percutaneous approach which allows to perform minimally invasive interventions. The catheters may help to perform, for example, a balloon angioplasty, stent placement, valve deployment, microsphere embolization, *etc.*

Successful passage of a catheter through the curves of the blood vessels requires not only a mastery of the art by the operator, but also a specific structure of the catheter which would ensure that the catheter can be manipulated and advanced in response to pushing and twisting of the proximal end of the catheter by the operator. US2010/268201 is a prior art document.

### SUMMARY

It is an object of the present disclosure to provide an accessory for a catheter for medical use for improving the performance characteristics of a medical device having such a catheter.

According to one aspect of the disclosed technology, there is provided a catheter accessory for use with a catheter according to claim 1. The dependent claims set out particular embodiments of the invention.

### BRIEF DESCRIPTION OF THE DRAWINGS

Further features and advantages of the present disclosure will become apparent from the following detailed description, taken in combination with the appended drawings, in which:
Fig. 1 depicts a delivery catheter with a catheter accessory, in accordance with at least one embodiment of the present disclosure;
Fig. 2 depicts the delivery catheter of Fig. 1 inserted into a guiding catheter and forming a catheter, in accordance with at least one embodiment of the present disclosure;
Fig. 3A schematically depicts the lateral cross-section of the distal portion and the intermediate portion of the catheter of Fig. 2, in accordance with at least one embodiment of the present disclosure;
Fig. 3B schematically depicts a side view of the catheter of Fig. 2 when a catheter accessory is in the operation mode, in accordance with at least one embodiment of the present disclosure;
Fig. 3C schematically depicts a side view of the catheter of Fig. 2 when a catheter accessory is in the mounting mode, in accordance with at least one embodiment of the present disclosure;
Figs. 4A-4C illustrate an embodiment of the catheter accessory of the present disclosure, which is implemented with a coil: a coiled accessory;
Fig. 5A depicts a mounting accessory, in accordance with at least one embodiment of the present disclosure;
Figs. 5B-5C depict portions of the mounting accessory of Fig. 5A;
Fig. 6A illustrates the process of mounting of the coiled accessory on the mounting accessory to put together the assembly, in accordance with at least one embodiment of the present disclosure;
Fig. 6B depicts the assembly of Fig. 6A while being mounted on the delivery catheter, in accordance with at least one embodiment of the present disclosure;
Fig. 7A illustrates the catheter accessory which has a stent-like structure - a stent-like accessory - in a collaped operating mode, in accordance with at least one embodiment of the present disclosure;
Fig. 7B illustrates the catheter accessory of Fig. 7A when mounted on the mounting accessory, in accordance with at least one embodiment of the present disclosure;
Figs. 8A-8D illustrate mounting of the stent-like accessory on the delivery stent, in accordance with at least one embodiment of the present disclosure of the present disclosure;
Figs. 9A-9C depict another embodiment of the catheter accessory- a side-opening accessory, in accordance with at least one embodiment of the present disclosure of the present disclosure;
Figs. 10A-10E depict another embodiment of the catheter accessory: an elastic expandable tubular element of the present disclosure;
Figs. 11A-11C depict another embodiment of the catheter accessory: a crimped closed-cell stent-like accessory of the present disclosure;
Figs. 12A-12C depict another embodiment of the catheter accessory referred to herein as a crimped open-cell stent-like accessory of the present disclosure;
Fig. 13 illustrates a back-end elongated element, in accordance with at least one embodiment of the present disclosure;
Fig. 14 schematically depicts another embodiment of the catheter accessory: a hybrid tubular accessory;
Figs. 15A-15B illustrates double-sheath accessory, in accordance with at least one embodiment of the present disclosure;
Figs. 16A - 16C illustrate perspective views of various portions of a coil-mesh hybrid tubular accessory, in accordance with at least one embodiment of the present disclosure;
Figs. 17A-17D illustrate coil-mesh hybrid tubular accessory of Figs. 16A-16C when installed on the delivery catheter, in accordance with at least one embodiment of the present disclosure;
Figs. 18A - 18C illustrate perspective views of various portions of a wave-like coiled accessory, in accordance with at least one embodiment of the present disclosure;
Figs. 19A-19D illustrate the wave-like coiled accessory of Figs. 18A-18C when installed on the delivery catheter, in accordance with at least one embodiment of the present disclosure; and
Figs. 20A-20E depict various non-limiting examples of a cross-sectional profile of the catheter accessory along the line 20-20 of Fig. 3A, in accordance with at least one embodiment of the present disclosure.

It will be noted that throughout the appended drawings, like features are identified by like reference numerals.

### DETAILED DESCRIPTION

Various aspects of the present disclosure generally address one or more of the problems of medical catheters.

There is described herein a catheter accessory for use with a catheter having a distal portion comprising an intervention accessory having a distal portion outer diameter. The catheter accessory comprises an elongated tubular structure, mounted onto the intermediate portion (it can be releasably mounted thereon for removing or interchanging the catheter accessory for different sizes or flexural rigidity parameters). However, the catheter accessory described herein, once installed onto a target position on the intermediate portion, is static during operation, as it does not move on the underlying catheter during operation. A material, shape and thickness are chosen to provide a pre-determined flexural rigidity and a pre-determined flexibility simultaneously to the intermediate portion covered by the elongated tubular structure, thereby giving the desired rigidity to the portion of the intermediate portion of the catheter covered by the catheter assembly. The elongated tubular structure has a default shape and size for operation with a lumen with an inside diameter larger than the intermediate portion outer diameter and smaller than the distal portion outer diameter, holding the catheter accessory onto the target location of the intermediate portion and being completely proximal to the distal portion comprising the intervention accessory (stent, balloon, etc.) during the operation, to be able to freely operate the intervention accessory at all times during operation by avoiding the catheter accessory covering partially or completely the intervention accessory which should protrude distally from the catheter accessory. For installation of the catheter assembly to its desired, static location, it can be slid from the proximal portion, in which case it needs to have an outer diameter smaller than an inside diameter of a lumen of a guiding catheter, and have a back-end elongated element to drive the sliding movement distally toward the target position and proximally back from that position for removal or interchangeability. Alternatively, the catheter accessory can be temporarily radially expandible (using an elastic material with suitable shape) to be inserted from the distal tip. In this case, the relatively large distal portion comprising the intervention accessory is slid through and across the lumen of the catheter accessory described herein, such that this catheter accessory reaches the desired static position on the intermediate, immediately but completely proximal the distal portion to keep the distal portion free to operate its intervention accessory while having the catheter accessory proximal thereto to rigidify the intermediate portion to provide the desired mechanical characteristics, especially to increase pushability from the proximal portion handled by the operator. The catheter accessory can be chosen with appropriate shape, thickness, material, or length for the task.

This differs from prior art devices, such as extension catheters, which may not be static during operation, and which can be expected to move onto the catheter or to cover at least partially or temporarily, during operation, the intervention accessory.

A conventional catheter has a hollow tube, a so-called guiding catheter, and a delivery catheter located therein. The conventional catheters vary in size and shape. The delivery catheter has a distal portion, which has a wire and a previously implanted device (such as, for example, a stent with a balloon), and a proximal portion. The operator manipulates proximal portions of the guiding catheter and of the delivery catheter located within the tube of the guiding catheter in order to advance the catheter through the blood vessels.

In operation, the guiding catheter is positioned to a close vicinity of a target which may be a stenosis, a tumor, a narrowed valve or a previously implanted device (such as, for example, a stent). Once the guiding catheter is positioned, the operator advances first a tiny wire (previously hidden inside the guiding catheter), which often vary from 0.356 mm to 0.889 mm (0.014' to 0.035') in diameter, to cross (pass by) the target or obstacle to be dilated or intervened. Once the wire is positioned across the lesion or stenosis, the operator slides, over the wire, a device required to perform the intended intervention.

During the medical intervention for a balloon angioplasty, the operator advances (moves forward) the delivery catheter which has a balloon with a stent, both in a collapsed state (the balloon and the stent in the collapsed state are referred also referred to herein as "collapsed balloon" and "collapsed stent", respectively). The collapsed balloon, together with the collapsed stent, is positioned at the location of a significant narrowed lesion (target). The operator pushes the balloon with the stent device across the stenosis until the operator is satisfied with the stent longitudinal position. In order to install the stent in the blood vessel, the operator inflates the balloon by inflating air from the proximal end until the stent is fully expanded (the balloon and the stent in the expanded positions are referred to herein as an "expanded balloon" and an "expanded stent", respectively), so that the initial lesion or blockage is removed, and the artery or the vessel regains a lumen (in other words, the inside space of the artery) which is larger than the passage across the target that was prior to the installation of the stent.

Due to anatomical variations or vessel tortuosities or calcifications, the operator might encounter difficulties in advancing the balloon, stent or other devices across the target lesion, stenosis or valve. To overcome such difficulties, the operator may try to use a catheter with a larger diameter.

However, increasing the diameter of the catheter might increase the risk of complications either at the entry site of the catheter into the blood vessel (e.g., radial or femoral access point) or inside the blood vessel where the distal portion of the catheter is located. Indeed, catheters with larger diameters might cause bleeding or vascular damages such as dissection.

In order to assist with the balloon and stent delivery, the shaft of the catheter needs to provide enough support, yet it has to remain significantly narrower than the catheter's widest diameter (which may be defined, for example, by the outer diameter of the collapsed balloon with the collapsed stent).

Various materials may be used to increase pushability of the catheter (the assembly of the guiding catheter and the delivery catheter) delivering the stent. However, currently available catheters sometimes are unable to cross the target lesion or an obstacle.

For example, a balloon angioplasty catheter with a monorail system has a distal portion of the catheter which is made of a soft, flexible plastic-type material and built as a hollow tube including a lumen for a wire and another lumen extending from the balloon to the proximal portion of the catheter. Such construction with two lumens allows an operator to inflate the balloon/stent using a syringe of a dedicated inflator device located at the proximal portion of the catheter. It should be noted that the material used for the proximal portion of the balloon angioplasty catheter with monorail system is usually more rigid and includes metallic part or a braided material to increase stiffness and hence pushability. The intermediate portion which includes two separate lumens and is located between the distal portion (having a stent or a balloon) and the proximal portion (for example, the intermediate portion may be located closer to the distal portion), is usually much softer than the proximal portion of the catheter. To increase the pushability of the catheter, it is desired to increase stiffness of the intermediate (monorail) portion of such catheter.

As referred to herein, the intermediate portion includes two separate lumens and is located between the distal portion (having a stent or a balloon) and the proximal portion (for example, the intermediate portion may be located closer to the distal portion.

As referred to herein, the pushability is a characteristic of a catheter which is related to a force (push force) needed to be applied by an operator (physician) to overcome a friction between the outer surface of the catheter and the interior walls of the blood vessels and to advance the catheter inside the blood vessels to the target. As the push force needs to be increased due to such friction, the catheter may start bending and twisting unnecessarily. The pushability of the catheter needs to be increased to reduce such unnecessary bending and twisting.

As referred to herein, a rigidity characterizes a degree of inability of the catheter or the catheter accessory to bend or to be forced out of shape. As referred to herein, a value of a flexural rigidity, EI, is determined as a Young's modulus multiplied by a bending moment of inertia.

A catheter accessory (which may also be referred to as an "enhanced sheath" or "sheath" or "stiffening sheath") described herein intends to increase stiffness and associated pushability of an intermediate portion of the catheter, which is located between the distal portion (carrying, for example, a balloon with a stent) and the proximal portion of the catheter. The catheter accessory as described herein is intended to increase stiffness of the intermediate portion of an existing delivery catheter (sometimes also referred to as a "stent catheter"), such as the balloon angioplasty catheter with monorail system. Described herein are various embodiments of the catheter accessory and methods of installation and use, which is not part of the invention, of the catheter accessory.

The catheter accessory described herein strengthens the catheter and, at the same time, ensures flexibility of the catheter. In addition, the catheter accessory is narrow enough to fit inside the guiding catheter or to fit in a blood vessel together with the delivery catheter (when no guiding catheter is used). In other terms, the catheter accessory described herein makes the catheter stiffer and may be referred to as stiffening catheter accessory. The catheter accessory as described herein may be installed by the operator via the distal portion of the catheter, by sliding over the distal portion of the delivery catheter as described herein below. The additional support provided by the catheter accessory may help in positioning of devices such as a balloon, a stent or a valve, for example, across the target lesion.

The catheter accessory as described herein enhances stiffening of the catheter, while maintaining a slender structure of the catheter, thus discouraging the operator to use a thicker catheter. The slender structure of the catheter is maintained because the catheter accessory as described herein is thin and has a diameter approximately equal to or narrower than the diameter of the distal portion of the delivery catheter. The catheter accessory as described herein may be also referred to a slender stiffening sheath which permits to increase a shaft support to a distal portion of the catheter. Such device aims to increase a shaft (intermediate's portion) stiffness to deliver balloon, stent, valve or other device for percutaneous intervention.

### Catheter accessory

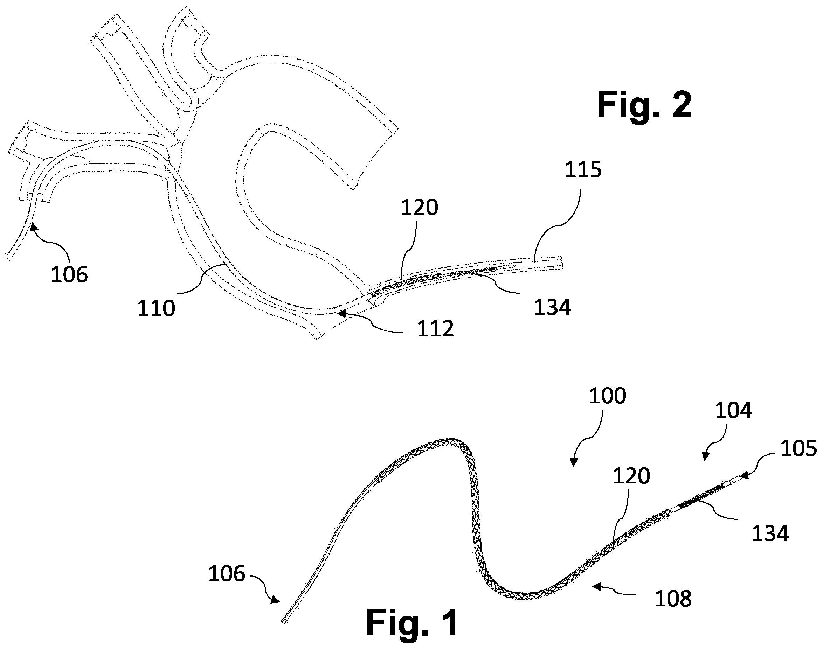

Fig. 1 depicts a delivery catheter 100 (also referred to herein as an "internal catheter" or a "stent catheter"), in accordance with at least one embodiment of the present disclosure. The delivery catheter 100 has a distal portion 104 ending with a tip 105, a proximal portion 106 which is closer to the practitioner and which is manipulated by the practitioner, and an intermediate portion 108. The intermediate portion 108 of the delivery catheter 100, especially close to the distal portion 104, is at least partially covered with a catheter accessory 120, in accordance with at least one embodiment of the present disclosure.

Fig. 2 depicts the delivery catheter 100 inserted into a guiding catheter 110 and forming a catheter 112. Fig. 2 illustrates the catheter 112 when inserted into the blood vessel 115. The guiding catheter 110 may coaxially cover the delivery catheter 100 and therefore hide inside the guiding catheter 110 at least partially, during the delivery of the devices, such as the collapsed stent and the collapsed balloon described above, to the target. The operator manipulates the proximal portion 106 of the delivery catheter 100 to move the whole catheter and to deliver and install, inside the blood vessel 115, the stent 134 with the balloon (not visible in Fig. 2 under the stent 134) which is originally provided at the distal portion 104 for this eventual delivery and installation.

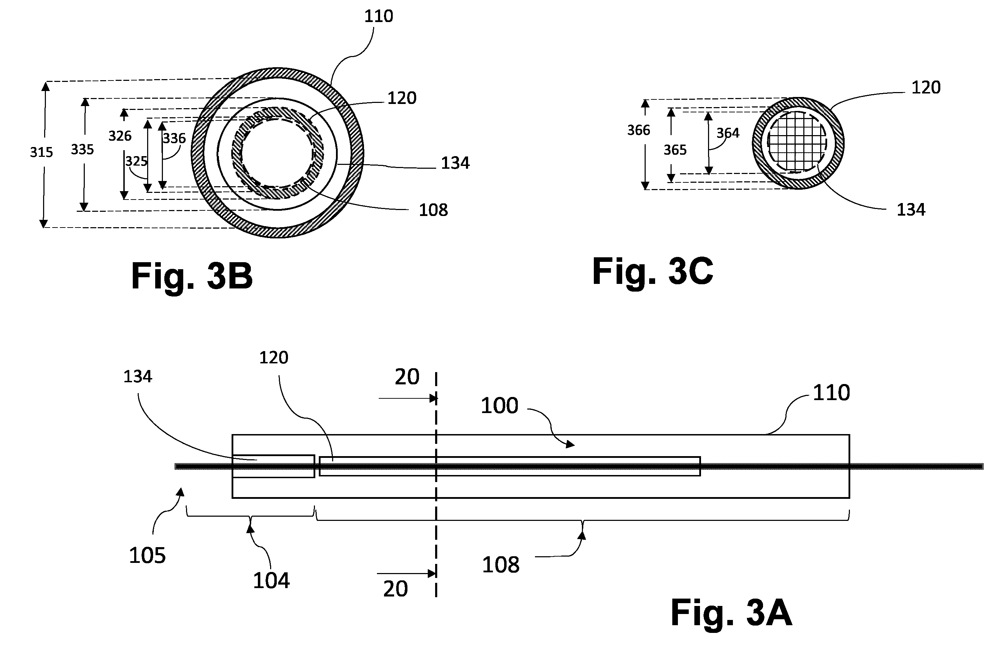

Fig. 3A schematically depicts the lateral cross-section of the distal portion 104 (including distal point or tip 105) and the intermediate portion 108 of the catheter 112, in accordance with at least one embodiment of the present disclosure. Fig. 3B schematically depicts a side view of the catheter 112 when a catheter accessory is in the operation mode, in accordance with at least one embodiment of the present disclosure. Fig. 3C schematically depicts a side view of the catheter of Fig. 2 when a catheter accessory is in the mounting mode, in accordance with at least one embodiment of the present disclosure.

The catheter accessory 120 as described herein is shorter than the delivery catheter 100 and does not cover the distal portion 104, or at least does not cover the tip 105. The catheter accessory 120 has a diameter which is not significantly larger than the diameter of the distal portion of the delivery catheter. Using the catheter accessory 120 should prevent the operator to use the guiding catheter 110 of larger diameter.

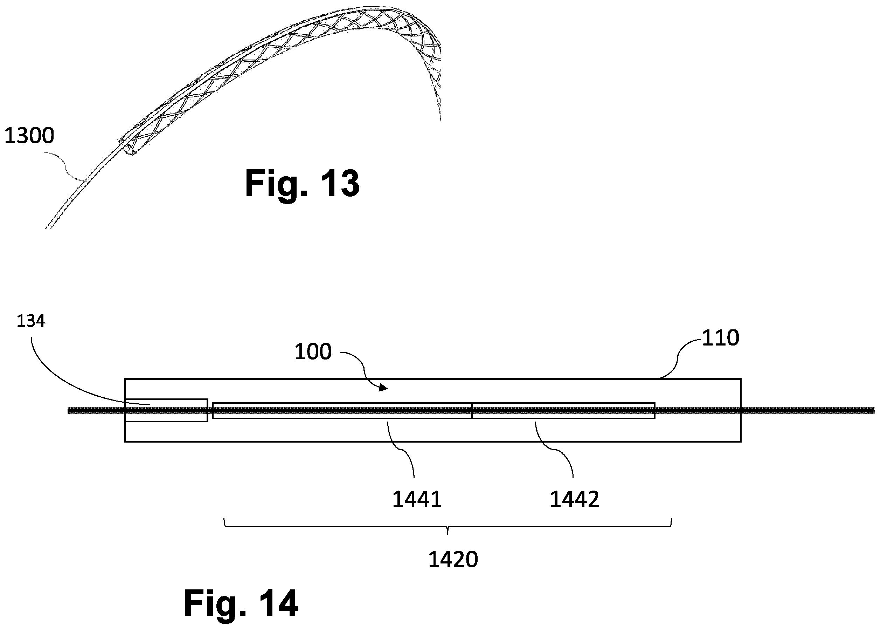

According to an embodiment, and described in further detail with respect to Fig. 13, the thin catheter accessory of variable length has a connecting wire (also described further below as the back-end elongated element 1300) so the operator may advance the catheter accessory while the delivery catheter remains inside the guiding catheter. Once the catheter accessory is slid toward the distal portion of the delivery catheter but proximal to the device itself such as balloon, stent or valve for example, the operator will benefit of increased support to advance the whole catheter device across the target lesion. After the balloon inflation and/or the stent delivery, the catheter accessory remains in position on the intermediate portion of the catheter and may be safely retrieved when the operator removes the catheter from the patient's body. During the same medical procedure, the catheter accessory may be removed from the catheter, as described below, and used with other delivery catheters provided they are of a similar size.

In some embodiments, the catheter accessory 120 as described herein has a circular cross-section.

The catheter accessory 120 described herein has an elongated tubular structure configured to coaxially removably mount on the intermediate portion 108, to attach to the intermediate portion 108 (for example, by gripping thereto) and provide a pre-determined partial rigidity and pushability, and a pre-determined flexibility simultaneously to the intermediate portion 108.

In use of the catheters in general, the operator usually would not have any possibility to install such catheter accessory 120 from the side of the proximal portion 106 of the catheter 112. The proximal portion 106 of the catheter is usually attached to other medical devices and, when an operator discovers that there is a need to install or to replace there is no possibility to detach the proximal portion 106 of the catheter. Therefore, the installation of the catheter accessory 120 as described herein would typically be performed from the catheter's distal portion 105 (or distal point or tip 105), from outside the catheter, then sliding into the proximal direction onto the tip 105 and onto the distal portion 104 until the sliding of the catheter accessory 120 to the desired position covering the intermediate portion 106, and not covering the distal portion 104 which typically comprises an accessory (stent, balloon, etc.). Therefore, for installation of the catheter accessory 120 coaxially with the delivery catheter 100 from the distal point 105, the accessory's lumen needs to be able to contain, temporarily, an inner diameter larger than the distal portion's outer diameter, which is defined by the outer diameters of the balloon, stent 134, or any other device installed in or on the distal portion 104 of the catheter. The diameter of the catheter accessory 120 may be increased by mechanically expanding the catheter accessory 120. Such expansion of the catheter accessory may be made, for example, with a mounting accessory as described herein below.

The elongated tubular structure of the catheter accessory 120 is configured to expand radially to a mounting mode having an expanded inner diameter larger than s distal portion's outer diameter and to collapse radially to an operation mode (gripping mode), in which it is secured tightly in place when located at the intermediate portion 108 of the catheter, since there is an expectation that during operation, the catheter accessory 120 will not move along the catheter and will not cover again the distal portion 104 which may contain an accessory (stent, balloon, etc.). Being able to contain such an accessory (stent, balloon, etc.) is only for the installation by sliding from the tip and not for operation, in which the catheter accessory 120 does not reach and does not cover such an accessory (stent, balloon, etc.). Referring to Fig. 3B, an inner operation diameter 325 of the catheter accessory 120 is equal to or larger than the outer intermediate portion diameter 336. An outer operation diameter 326 of the catheter accessory 120 is equal to or smaller than the outer distal portion diameter 335, in order to fit within the guiding catheter 110 and its internal diameter. It should be noted that the difference between the outer operation diameter 326 of the catheter accessory 120 and the inner operation diameter 325 of the catheter accessory 120 is referred to herein as a "sheath thickness" of the catheter accessory 120. The sheath thickness of the catheter accessory 120 may be between about 0.1 millimeter (mm) and about 3mm.

The catheter accessory 120 as described herein may have various cross-sectional profiles (of the stiffening component) such as, for example: rectangular, circular, elliptical or polygonal. Various cross-sectional profiles determine a bending moment of inertia properties and thus provide different levels of rigidity.

In at least one embodiment, the catheter accessory 120 has one or more structural components that form a full or a partial sheath and provide increased axial and flexural rigidity due to a combination of geometric features along with the mechanical properties of the material from which the catheter accessory 120 is made.

The flexural rigidity El (determined as a Young's Modulus multiplied by a bending moment of inertia) of the catheter accessory 120 may be, for example, between approximately 0.0001 Pascal multiplied by meter⁴ (Pa m⁴) to approximately 0.002 Pa m⁴. Such a flexural rigidity provides suitable flexibility to the catheter for manipulation on one side, and sufficient pushability for pushing the catheter forward. It should be mentioned that the pushability of the catheter depends on the flexural rigidity of the catheter accessory 120 which defines the flexural rigidity of the intermediate section of the catheter when the catheter accessory 120 is installed thereon.

The surface texture of the catheter accessory 120 is such that there is no or little friction when the catheter accessory 120 moves over the other devices of the delivery catheter during the installation. When the catheter accessory 120 is installed on the intermediate portion 108, the texture of the outer surface of the catheter accessory 120 is such that the friction with the inner surface of the guiding catheter is minimized during the operation.

The distal and proximal ends (in other words, end portions or tips) of the catheter accessory 120 may have a chamfered edge, or a conical-shape feature, or a rounded edge, or a smooth edge created by electropolishing process, or a protective cover in the form of a coating, or a protective component attached to either tip of the catheter accessory 120 to avoid any potential damage to the vessels when in clinical use.

The length of the catheter accessory 120 may be between about 10 centimeters (cm) and about 30 cm, preferably between about 15 cm and 25 cm, and more preferably of about 20 cm. The outer diameter of the catheter accessory 120 may be equal to or smaller than about 8.0 mm. The inner diameter of the catheter accessory 120 may be equal to or larger than 0.35 mm.

### Coil

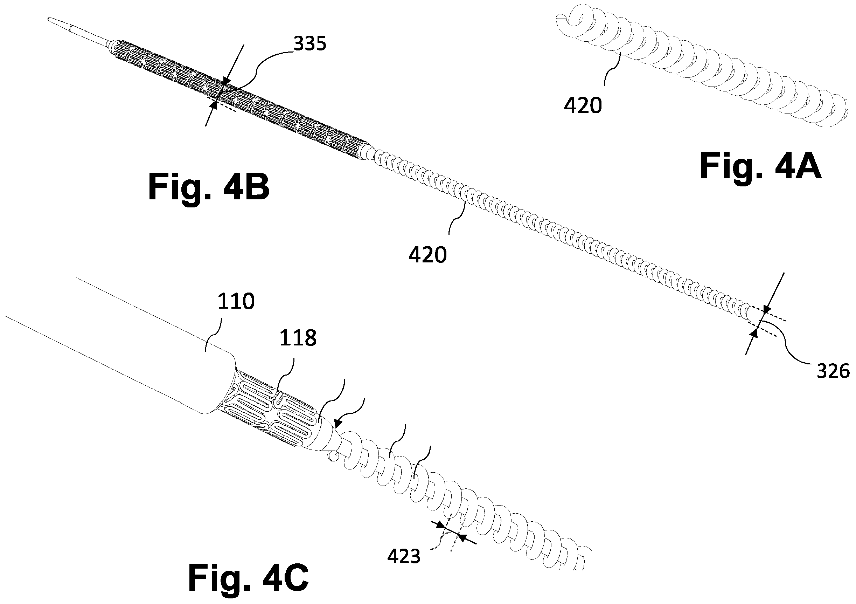

The catheter accessory 120 may have a spiraling structure. Fig. 4A illustrates an embodiment of the catheter accessory 120 of the present disclosure, which is implemented with a coil: the coiled accessory 420. In some embodiments, the coiled accessory 420 may be a spring coil.

The coiled accessory 420 comprises a tubular (cylindrical) element having a shape of a coil made of a single or a plurality of wires made of a medically suitable metallic or polymeric material and provided in the form of a tube. For example, and without limitation, the spiraling structure may have a circular cross-section profile or a rectangular profile cross-section.

Figs. 4B, 4C illustrate the coiled accessory 420 installed on the intermediate portion of the delivery catheter 100.

The structural rigidity of the coiled accessory 420 is determined by a pitch 423, a material, thickness, and cross-sectional profile of the coiled accessory 420. The coiled accessory 420 has a spiraling structure of constant or variable radius, constant or variable pitch and constant or variable thickness or profile in one end of the device.

The pitch (also referred to herein as a "gap distance") of the coiled accessory 420 may be adjusted either by compressing the spiral longitudinally using a tension cable located inside the spiral or by the twisting motion of the coiled accessory 420. Both methods would permit the operator to control and adjust the stiffness of the intermediate portion 108 of the catheter 112.

As for other embodiments of the catheter accessory 120, flexural rigidity of the coiled accessory 420 (Young's modulus multiplied by a bending moment of inertia) El may be between about 0.0001 Pa m⁴ and about 0.002 Pa m⁴.The pre-determined pitch 423 and thickness of the structural spiraling component determines the level of stiffness (in other terms, the value of stiffness) and/or rigidity that is added to the catheter's initial stiffness and initial rigidity.

The outer diameter 326 of the coiled accessory 420 when it is in the collapsed mode is such that the coiled accessory 420 may fit inside the guiding catheter 110 and move within the guiding catheter 110 without friction, or with a low level of friction.

The inner diameter of the coiled accessory 420 may be slightly larger than the outside diameter of the inner component which would need to pass therethrough during installation, and/or it may have a material or coating chosen for its suitable tribological qualities (as described below), such that it reduces the friction levels between the element and the inner component such as stent catheter. In some embodiments, the inner diameter may touch the stent catheter and thus has an increased friction level. The coiled accessory 420 may be made of metal alloys such as stainless steel, nitinol, cobalt-chrome or platinum-chrome. Alternatively, the coiled accessory 420 may be made of polymer-based materials.

The coiled accessory 420 may be coated with a coating, for example, with a hydrophilic or a hydrophobic polymeric coating to adjust the friction levels. The coating may have anti-thrombotic properties to reduce coagulation or clotting effects, for example it can be coated with heparin.

The length of the coiled accessory 420 may be between about 10 cm and about 30 cm, preferably between about 15 cm and 25 cm, and more preferably of about 20cm. The diameter of coil wire of the coiled accessory 420 may be between about 0.1 mm to about 3 mm. The pitch of coil wire may be between about 0.1mm to about 5 mm.

### Installation

According to an embodiment of the disclosure, for passing the distal portion 104 of the delivery catheter 100 through the lumen of the coiled accessory 420, the coiled accessory 420 has a mechanical property (combination of material and default shape) of being capable to be temporarily expanded radially (elastic deformation from the default shape to an expanded shape, and back again to the default shape thanks to the elasticity of the coiled accessory 420). Such expanding of the coiled accessory 420 may be performed by torsioning the coil (spiral).

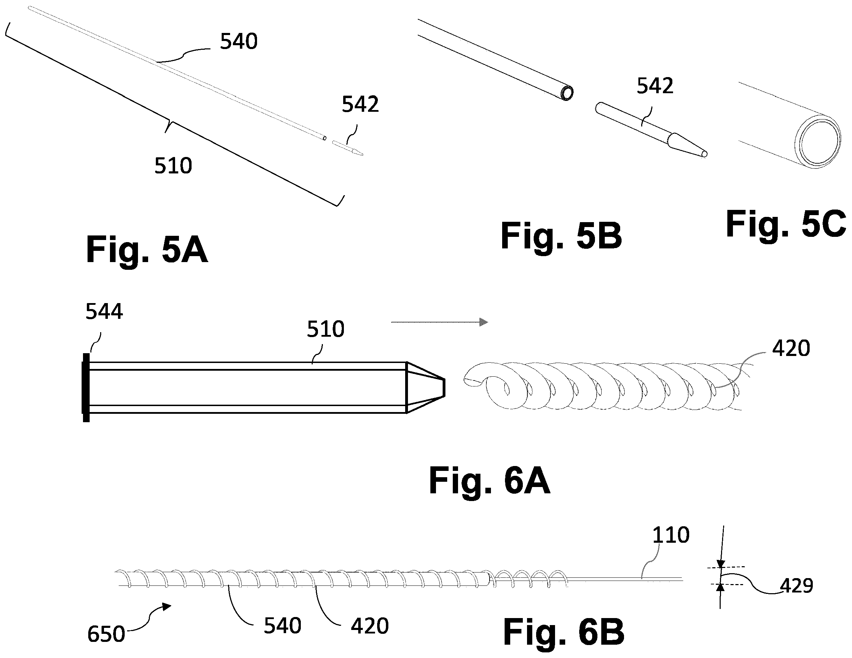

Alternatively, the coiled accessory 420 may be expanded to mount the coiled accessory 420 onto the delivery catheter 100. The coiled accessory 420 may be mounted coaxially on the delivery catheter 100 by using a mounting accessory such as, for example, a mandrel in the shape of a tube. Fig. 5A depicts the mounting accessory 510, and Figs. 5B, 5C depict the portions of the mounting accessory 510, in accordance with at least one embodiment of the present disclosure.

The mounting accessory 510 may be made of metallic alloys such as stainless steel, nitinol or made of a polymeric material such as ABS, Nylon, HDPE, PC. This mounting accessory 510 is longer than the coiled accessory 420 thus extending the length of the assembly which includes the mounting accessory 510 and the coiled accessory 420.

The mounting accessory 510 expands the cross-section diameter of the coiled accessory 420 along an entire length of the coiled accessory 420. The mounting accessory has an inner diameter large enough to cross (pass through) the stent structure in its crimped form (that is the retracted form of the stent in its smallest diameter). The outer diameter of the mounting accessory is such that allows for the temporary expansion of the coil element in an elastic deformation state by forcing a radial extension from the inside of the lumen outwardly starting from the insertion point of the mounting accessory 510 into the lumen of the coiled accessory 420.

The mounting accessory 510 (also referred to herein as a "mandrel") has a tube 540 with a tip 542. The tip 542 has at least partially a cone or a truncated cone with the smaller tip diameter being smaller than the internal diameter of the coiled accessory 420. As depicted in Figs. 5B, 6A, the mounting accessory 510 has a conical shape portion at one end to facilitate insertion of the mounting accessory 510 into the coiled accessory 420. The mounting accessory 510 may also have a stopper portion 544 at the opposite end to prevent the coiled accessory 420 from overcrossing the coiled accessory 420.

The coiled accessory 420 may be installed (mounted) on the delivery catheter 100 from the distal point 105 by expanding the coiled accessory 420 to a mounting mode.

To mount the coiled accessory 420 onto the delivery catheter 100, the operator inserts the mounting accessory 510 into the coiled accessory 420 with the help of the conical shape of the mounting accessory tip 542 in one end of the mounting accessory 510. The mounting accessory tip 542 expands the coiled accessory 420 which increases the diameter temporarily because of an elastic deformation allowed by the material of the coiled accessory 420 as well as its coil shape which, combined to the material, allows such an elastic deformation. As the coiled accessory 420 expands under the force applied from inside outwardly by the mandrel or mounting accessory 510, it is slidden throughout the entire length of the mounting accessory 510 until the coiled accessory 420 reaches the stopper in the opposite end of the mounting accessory, thus forming the assembly 650 (Fig. 6B).

Fig. 6A illustrates the process of mounting of the coiled accessory 420 on the mounting accessory 510 to put together the assembly 650. Fig. 6B depicts the assembly 650 while being mounted on the delivery catheter 100, in accordance with at least one embodiment.

Once the coiled accessory 420 has been expanded, the operator passes the delivery catheter (for example, the stent catheter) through the internal hole of the mounting accessory 510 until the stent and balloon (or other devices located in the distal portion of the catheter) pass beyond the length of the assembly 650. To deploy the coiled accessory 420 on top of the intermediate portion 106 of the delivery catheter 100, the operator retains the coiled accessory 420 by hand and then slide away the mounting accessory 510. This allows the coiled accessory 420 to collapse back into its intended stress-free original dimension and shape (or default size and shape), which is referred to herein as "operation mode" of the coiled accessory 420. The coiled accessory 420 in the operation mode needs to fit inside the space of the guiding catheter 110 when in clinical use.

In the mounting mode of the coiled accessory 420, an inner expanded diameter 365 of the coiled accessory 420 is larger than the outer distal portion diameter 364 (see Fig. 3C), which is determined by the largest outer diameter of the stent and the balloon, or any other device located at the distal portion of the delivery catheter 100. When in the mounting mode, the coiled accessory 420 can be mounted through the distal point 105 of the delivery catheter 100 by passing the distal portion 104 of the delivery catheter 100 through the inlet (tube, cavity) of the coiled accessory 420 coaxially, or approximately coaxially. As described above, it may be impossible for the operator to mount the coiled accessory 420 from the proximal portion 106 of the delivery catheter 100.

The expansion of the coiled accessory 420 allows for the mounting of the coiled accessory 420 on the delivery catheter 100.

The inner diameter 429 of the coiled accessory 420 when mounted on the mounting accessory 510 (referred to herein as an inner expanded diameter and outer expanded diameter) is larger than the diameter 326 of coiled accessory 420 when the coiled accessory 420 is collapsed (and has an inner collapsed diameter and an outer collapsed diameter).

When the coiled accessory 420 is being installed using the mounting accessory 510 (mandrel), the inner diameter of the assembly 650 is maintained by the mandrel 510 and the inner diameter of the assembly 650 is determined by the outer diameter of the mandrel 510. To install the accessory with the mandrel, the assembly's inner diameter needs to be larger than the distal portion's outer diameter 364 in order to be able to pass through the distal portion of the delivery catheter, which has a balloon, stent, or other devices mounted thereon.

The temporary expansion of the coiled accessory 420 using the mandrel allows for its elastic deformation but avoids plastic deformation. In other terms, the deformed (expanded) coiled accessory 420 does not stay in the expanded state, but rather, as soon as the mandrel is removed, the coiled accessory 420 collapses radially to an operation mode (depicted in Fig. 4B) with default size and shape. This allows for the coiled accessory 420 to collapse back into its original stress-free diameter for its intended clinical use on top of the delivery catheter 100 and inside the guiding catheter 110, and especially not covering the distal end 104 with the intervention accessory that is expected to be found on the distal end 104 (stent, balloon, etc.). In other words, the coil of the coiled accessory 420 is configured to temporally expand radially, in response to a force applied radially (for example, by the mandrel), by the elastic deformation and to increase a diameter of a tubular opening therein, and should extend, at its own distal, to a distal limit which is proximal to the distal end 104 with the intervention accessory, thereby not covering the intervention accessory, and being static onto the delivery catheter 100 during operation. On the other hand, when the force is not applied radially (for example, by the mandrel), the coil collapses and decreases the diameter of the tubular opening back to its original stress-free diameter.

Referring also in Fig. 3B, in the operation mode, the inner operation diameter 325 of the coiled accessory 420 (which the embodiment of the catheter accessory 120) is larger than the outer intermediate portion diameter 336. The outer operation diameter 326 is equal to or smaller than the outer distal portion diameter 335 in order to follow the distal portion and not unnecessarily disturb the walls of the vessels.

It should be noted that the mounting accessory 510 may also be used to mount the other embodiments of the catheter accessory 120. The mounting accessory 510 may also be used to remove the catheter accessory 120 from the delivery catheter 100 by the operator. For example, the operator may want to use another catheter accessory 120 with the same delivery catheter 100 in order to increase or decrease flexibility of the catheter or increase or decrease the rigidity of the catheter, either sequentially (one being distal to the other one to forms a sequential series of two, three, etc. or more accessories 120 placed relatively distally or proximally from each other and not overlapping) or concentrically (a second, larger one around the first, narrower one).

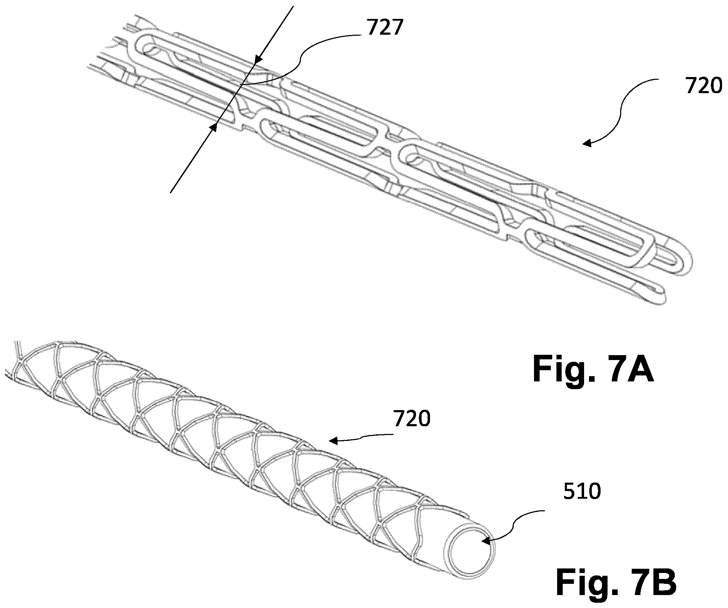

The opposite end, i.e., most proximal portion of the device comprises a long and constant profile element that extends beyond the length of the typical guiding catheters so that the physician can manipulate the device by pulling, pushing or torsioning the device while performing cardiovascular interventional procedures. This long element can be attached to the coil element by means of welding, crimping, gluing, mechanical lock or simply by being part of the same wire element as to form a single piece device. It should be noted that other embodiments of the catheter accessory 120 described herein (with reference to, for example, Figs. 7A-19D) may also have such a long and constant profile element with similar characteristics and functions.

### Self-collapsing stent-like structure

In at least one embodiment, the catheter accessory 120 may have a stent-like structure. This embodiment is referred to herein as a stent-like accessory 720 and is illustrated in Fig. 7A in a collapsed operating mode. Fig. 7B illustrates the stent-like accessory 720 when mounted on the mounting accessory 510, in accordance with at least one embodiment of the present disclosure. The stent-like accessory 720 has geometrical features that resemble a closed-cell stent structure that expands radially with relative ease. The structural rigidity of this stent-like accessory 720 is defined by the material, strut thickness, and the different geometrical features of the stent-like structure such as crowns and flexing bridges. The stent-like accessory 720 may be made of a shape memory alloy such as, for example, nitinol or any other metallic or polymeric material. The memory alloy exhibits a mechanical behavior of high yield stress before plasticity thus a high deformability in its elastic zone.

The outer diameter 727 of the stent-like accessory 720 is such that it allows for the relative motion of the stent-like accessory 720 against the guiding catheter 110 with low friction when it is inserted therein. As discussed above with respect to another embodiment, the inner diameter may be such that the friction between the stent-like accessory and the intermediate portion of the stent catheter is low. In some other embodiments, the inner diameter may be such that the stent-like accessory 720 touches the intermediate portion 108 of the delivery catheter 100 located inside (coaxially or otherwise) and thus rub against each other (in other terms, has an increased friction level therebetween).

The stent-like accessory 720 may be coated with a hydrophilic or a hydrophobic polymeric coating to adjust the friction levels of the stent-like accessory 720. The coating may have anti-thrombotic properties to reduce coagulation or clotting effects. The stent-like accessory 720 may have dimensions similar to those described above for the coiled accessory. Similar to the coiled accessory 420, the stent-like accessory 720 has the mechanical property to expand to a larger diameter and to collapse to a smaller diameter.

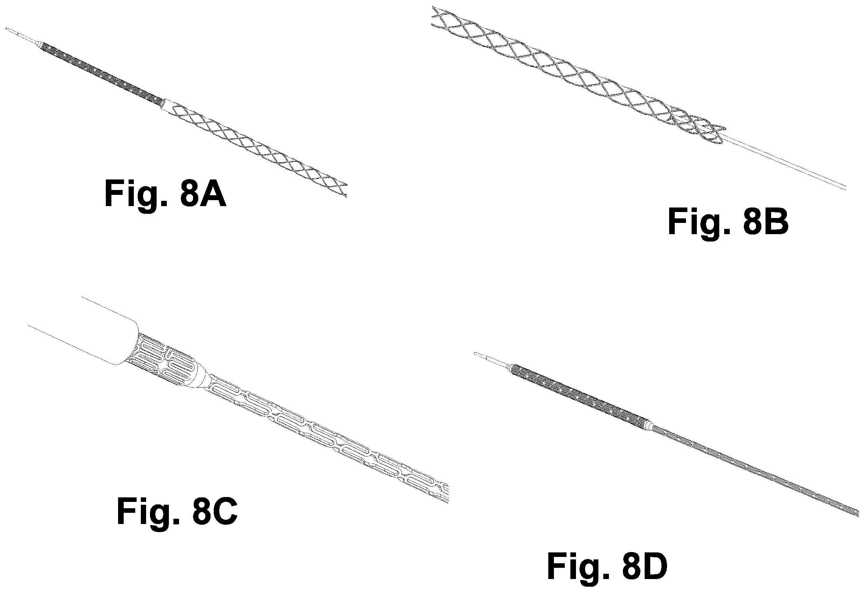

Figs. 8A-8D illustrate mounting of the stent-like accessory 720 on the delivery stent 100. The stent-like accessory 720 may be installed using the mounting accessory 510 as described above in a similar manner as the coiled accessory 420 is mounted on the intermediate portion 108 of the catheter as described above. Thus, the stent-like accessory 720 may expand using the mounting accessory 510 and allow for elastic expansion (deformation), without plastic deformation. This permits the stent-like accessory to collapse back to its original stress-free shape with a smaller diameter to fit within the guiding catheter 110 when in clinical use.

Strut thickness of the stent-like accessory 720 may be between about 0.1 mm and about 3 mm. The stent-like accessory 720 may have a plurality of interconnecting struts or bridges along its longitudinal axis between the crown-like patterns to modulate the overall flexural stiffness.

### Side-opening self-collapsing structure

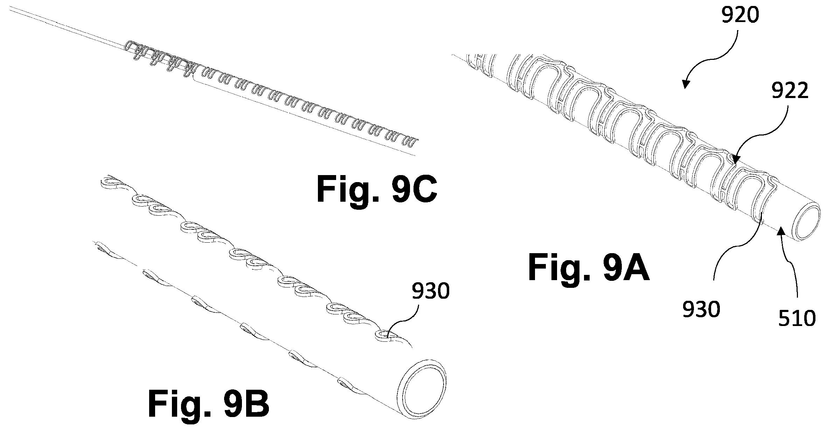

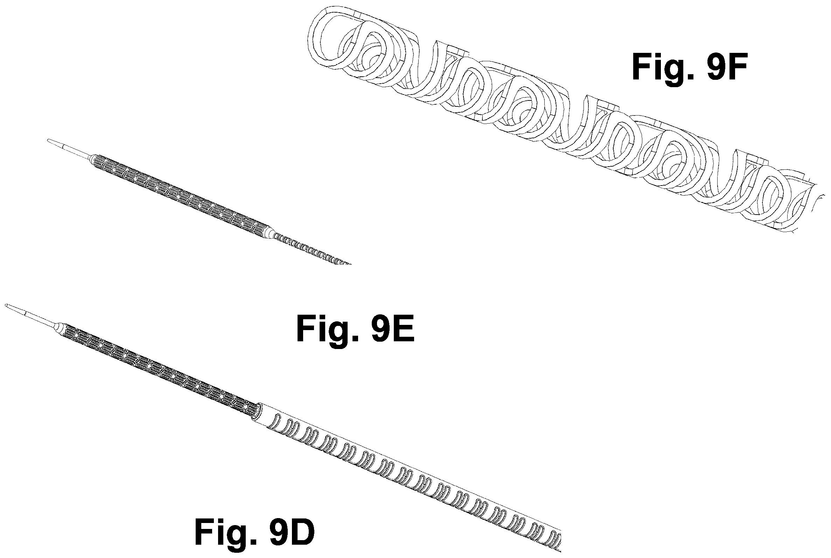

Fig. 9A-9C depict another embodiment of the catheter accessory 120 - the side-opening accessory 920 which has a side opening to allow for lateral installation of the side-opening accessory 920. The geometrical features of this the side-opening accessory 920 have circular open-end arms 930 originating from a single backbone element 922 (formed, for example, by the accessory wires). The backbone geometry of the side-opening accessory 920 may be straight or in a helical pattern to accommodate all open-end arms 930.

The structural rigidity of this side-opening accessory 920 is determined by the material, arm thickness, arm geometry and the length of the arms. The side-opening accessory 920 may be made of a shape memory alloy such as nitinol or any other metallic or polymeric material which exhibits a mechanical behavior of high yield stress before plasticity thus a high deformability in its elastic zone.

The installation of the side-opening accessory 920 is similar to the installation of the stent-like accessory 720 and the coiled accessory 120. The side-opening accessory 920 may be installed on the intermediate portion of the delivery catheter using the mounting accessory 510 as described above. When the mounting accessory 510 is pulled out from the side-opening accessory 920, the side-opening accessory collapses from the mounting mode (illustrated in Figs. 9A-9D) to the collapsed operation mode (illustrated in Figs. 9E-9F). The installation procedure of the side-opening accessory 920 is the same as described above for the stent-like accessory 720. Therefore, the relations between the diameters of the distal portion of the delivery catheter, intermediate portion of the delivery catheter, and diameters of the stent-like accessory 720 in the mounting mode and the operation mode as described above are applicable to the diameters of the side-opening accessory 920. Such side-opening accessory 920 may also have a coating as described above for the stent-like accessory 720.

The side-opening accessory 920 may have a plurality of interconnecting struts or bridges along its longitudinal axis between the crown-like patterns to modulate the overall flexural stiffness. The length of each circumferential arm may extend in its circumferential length from 50% up to 95% of the of circumference of the tubular structure of the side-opening accessory 920. Flexural rigidity (Young's modulus multiplied by a bending moment of inertia) El may be between about 0.0001 Pa m⁴ and about 0.002 Pa m⁴.

### Elastic Expandable tube

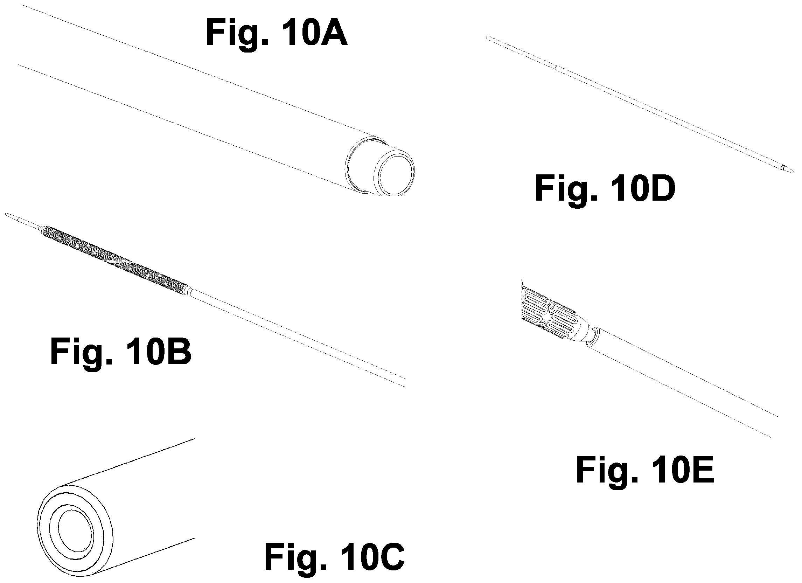

Figs. 10A-10E depict another embodiment of the catheter accessory 120: an elastic expandable tubular element 1020 which is tubular (has a shape of a tube) and is made of an elastomeric material. Figs. 10A-10E also illustrate the stages of installation of the elastic expandable tubular element 1020 using the mounting accessory 510 described above by forming an assembly first and then passing distal portion of the delivery catheter through the hollow cavity of such assembly. The description of installation of the other embodiments of the catheter accessory 120 applies also to the installation of the elastic expandable tubular element 1020.

The mechanical properties of the material, from which the elastic expandable tubular element 1020 is made, allow it to radially expand by a significant amount of deformation or strain without causing any permanent deformation or plasticity.

The structural rigidity of the elastic expandable tubular element 1020 is defined by the material properties, thickness, and length of the elastic expandable tubular element. The elastic expandable tubular element 1020 may be made of an elastomeric material such as silicone rubber or thermoplastic urethane or any other polymeric material that exhibits a mechanical behavior of high yield stress before plasticity thus a high deformability in its elastic zone. The elastic expandable tubular element 1020 may be mounted similarly to other embodiments of the catheter accessory 120 as described herein.

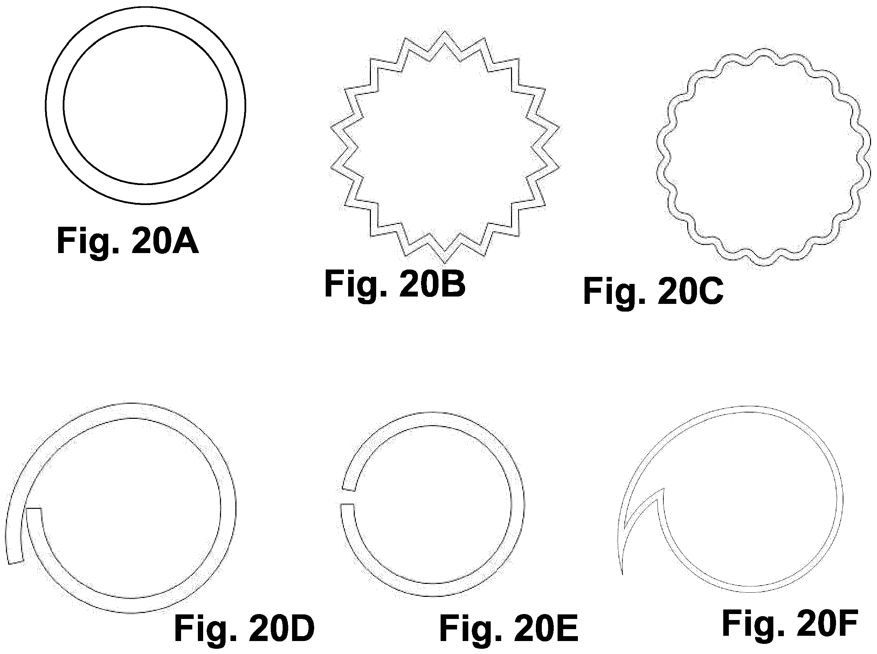

Figs. 10-10A depict a solid elastomeric tube embodiment of the elastic expandable tubular element 1020 which has a solid (round) profile of its cross-section as depicted in Fig. 20A. The elastic expandable tubular element 1020 may also have other cross-sectional profiles as depicted in Figs. 20B-20F. The elastic expandable tubular element 1020 may be also referred to as an "elastic expandable tube" with an extruded tubular structure that may be expanded to a larger diameter with the purpose of crossing the stent (or another device) from the distal end of the catheter in order to place the elastic expandable tubular element 1020. Polymeric materials are preferred in this embodiment although metallic materials may be also used. The preferred materials for elastic expandable tubular element 1020 are elastomeric polymers such as silicone rubber or thermoplastic urethane or any other polymeric material, thus forming an elastomeric tubular element.

In some embodiments, the elastic expandable tubular element may expand also due to their geometrical features, in addition to (or, instead of, if the materials are metallic) the elastic properties of the materials. The cross-sectional profile (along the lines 20-20 of Fig. 3A for the catheter accessory) of elastic expandable tubular element 1020 may be unsmooth or draped (folded) such that the expansion and collapse is help by the geometry of the tubular structure. In at least one embodiment, the elastic expandable tubular element 1020 may have a star-like uniform extruded cross-sectional profile as illustrated in Fig. 20B. The wavy pattern of the star-shaped profile is intended to increase the radial elastic capability of the tubular structure. Such draped cross-sectional profile may enhance the radial elasticity (elastic properties and expansion properties) of the elastic expandable tubular element. For this embodiment, the star-shaped profile may be a conventional star with sharp angled peaks as illustrated in Fig. 20B or a lobular star with soft corners (Fig. 20C) (in other terms, unshaped peaks, providing a wave-like profile). The number of peaks in this star-shaped profile (conventional or lobular) may range from 3 peaks up to 64 and their respective internal and external diameter should fall within the dimensions for the intended clinical application and as described herein.

Figs. 20D, 20E depict various non-limiting examples of a cross-sectional profile of another embodiment of elastic expandable tubular element 1020 which is an open-end tube with a uniform extruded profile to allow for radial elastic expansion. This open-end nature of such embodiment allows it to be positioned from the side instead of inserting it from the distal part of the catheter. The open-end tubular structure may have a cross-sectional profile where the open circumferential edges meet when the structure is closed (for example, providing a gap therebetween as depicted in Fig. 20D), or it may have a geometry where the open circumferential edges overlap with each other when in the closed position as depicted in Fig. 20E.

Fig. 20F illustrates a cross-sectional view of another embodiment of the elastic expandable tubular element 1020 with has an irregular-shaped closed tube with a uniform extruded profile to allow for radial elastic expansion. Such pleated tubular structure may be expanded radially to be able to insert it from the distal portion of the catheter because its geometrical features allow it to unfold to a greater diameter. Once the tubular structure is in the final position on the catheter, the structure is folded back to collapse onto the intermediate portion of the delivery catheter. An example of this type of irregular shape would be a "pleated" cross-section of the elastic expandable tubular element 1020 in which the number of pleats may range from 1 to 16. Such a pleated cross-sectional profile with at least one pleat located along the length of the elastic expandable tubular element enhances a radial elasticity of the elastic expandable tubular element.

The shore hardness of the elastic expandable tubular element 1020 may range from Shore A 30 to Shore A 90. The inner and outer surfaces of the elastic expandable tubular element 1020 may be coated with a low-friction surface to allow for easy gliding of the elastic expandable tubular element 1020. Flexural rigidity (Young's Modulus x Bending Moment of Inertia), also known as EI (where E is the flexural modulus (in Pa), I is the second moment of area (in m⁴), may be between about 0.0001 Pa m⁴ and about 0.002 Pa m⁴.

### Crimped closed-cell stent-like accessory

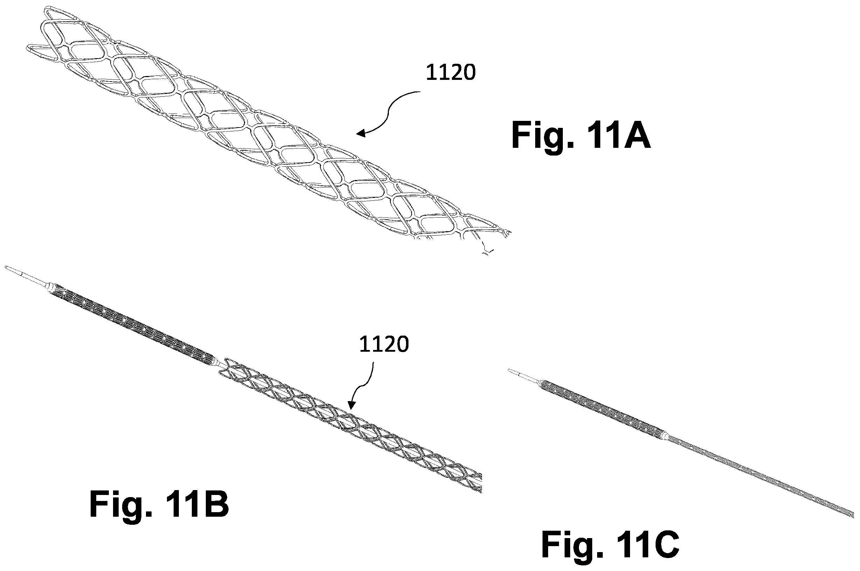

Referring now to Figs. 11A-11C, where another embodiment of the catheter accessory 120 is depicted: a crimped closed-cell stent-like accessory 1120. The crimped closed-cell stent-like accessory 1120 has an elongated tubular structure. The crimped closed-cell stent-like accessory 1120 may be made of a metallic or polymeric material. This crimped closed-cell stent-like accessory 1120 has a closed-cell stent-like structure that has a geometric pattern similar to a pattern of a stent. The crimped closed-cell stent-like accessory 1120 is collapsible when a force is applied to its walls along its length. The crimped closed-cell stent-like accessory 1120 may be manufactured in an expanded state with an internal diameter large enough to fit and cross (pass over) a coronary stent crimped on top of a balloon catheter, or another device located in the distal portion of the delivery catheter.

The crimped closed-cell stent-like accessory 1120 may have a plurality of interconnecting struts or bridges along its longitudinal axis between the crown-like patterns to modulate the overall flexural stiffness.

### Crimped open-cell stent-like accessory

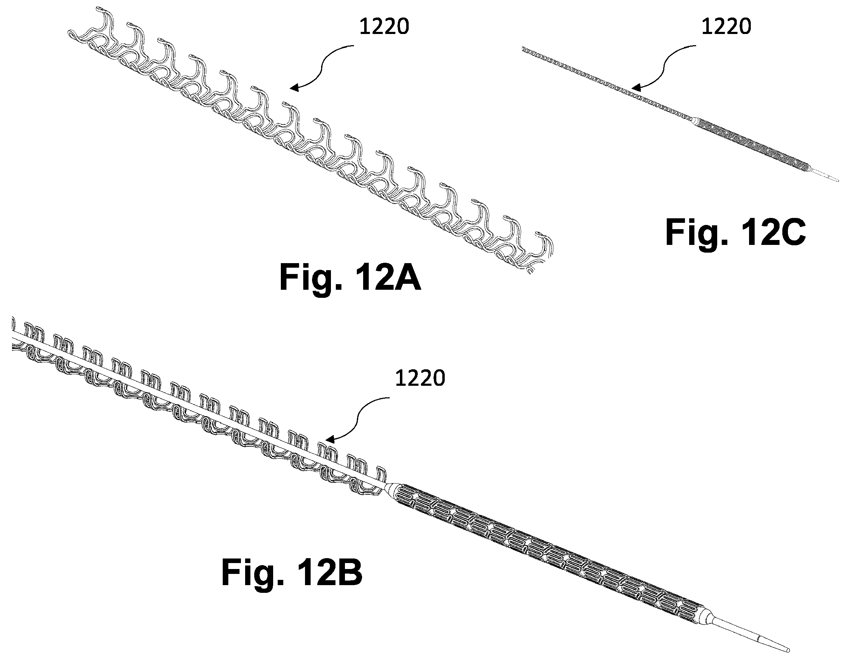

Figs. 12A-12C depict another embodiment of the catheter accessory 120 referred to herein as a crimped open-cell stent-like accessory 1220. The crimped open-cell stent-like accessory may be made of a metallic or a polymeric material. This crimped open-cell stent-like accessory 1220 may have an open-cell or open-end stent-like structures that have a geometric pattern typical of an open-end stent. The crimped open-cell stent-like accessory 1220 is collapsible. The stent-like accessory 1120 is designed and manufactured in an expanded state with an internal diameter large enough to fit and pass through (cross) the intervention accessory on the distal end 104, such as a coronary stent crimped on top of the balloon catheter, during installation until it reaches its final, static position onto the intermediate portion 106, immediately proximal to the distal end 104 comprising the intervention accessory and not covering it anymore. The tubular elements length feature may be, for example, between approximately 10 cm and approximately 30 cm.

The material mechanical properties allow the crimped closed-cell stent-like accessory 1120, open-cell stent-like accessory, and open-end stent-like accessory 1220 to be deformed significantly in the plastic (and not elastic) range of deformation to force the collapsing of the closed cell stent-like structure, open-cell stent-like accessory, and open-end stent-like accessory on top of (around or partially around) the intermediate portion of the delivery catheter 100 by radial crimping process in a permanent way. The crimping process forces the crimped closed-cell stent-like accessory 1120, open-cell stent-like accessory, and open-end stent-like accessory 1220 to collapse onto the delivery catheter 100 by means of mechanical deformation and strain.

The material for these embodiments of the accessory - crimped closed-cell stent-like accessory 1120, open-cell stent-like accessory, and open-end stent-like accessory 1220- may be stainless steel, cobalt-chrome or platinum-chrome or any other polymeric or metallic material capable of withstanding mechanical strains above 5% in a stress-strain curve without fracture. The flexural structural rigidity of the crimped closed-cell stent-like accessory 1120, open-cell stent-like accessory, and open-end stent-like accessory 1220 may be determined by the material properties, thickness, and lengths of the crimped closed-cell stent-like accessory, open-cell stent-like accessory, and open-end stent-like accessory, respectively.

The original inner diameter of the crimped closed-cell stent-like accessory and open-cell stent-like accessory before the crimping process is such that it allows the crimped closed-cell stent-like accessory to pass (cross) via the distal portion (from the distal point) in order to mount the crimped closed-cell stent-like accessory (or open-cell stent-like accessory) on the intermediate portion of the catheter. As mentioned above, the diameter of the distal portion of the catheter may be defined by the outer diameter of the crimped stent which is already mounted on the balloon of the stent catheter, or by the outer diameter of another device mounted on the distal portion of the catheter.

The original outer diameter is such that once the crimped closed-cell stent-like accessory has been crimped, the crimped closed-cell stent-like accessory fits inside the space between the inner diameter of a guiding catheter (for example, a 5F guiding catheter) and the outer diameter of the intermediate portion (guiding section) of the stent catheter.

The outer diameter of the accessories (crimped closed-cell stent-like accessory, open-cell stent-like accessory, or open-end stent-like accessory) after the crimping process allows for the relative motion of the device against the guiding catheter without friction or with a low friction. The inner diameter may be such that it reduces the friction levels between the accessory (crimped closed-cell stent-like accessory, open-cell stent-like accessory, or open-end stent-like accessory) and the inner component such as stent catheter. In some embodiments, the inner diameter of the accessory (crimped closed-cell stent-like accessory, open-cell stent-like accessory, or open-end stent-like accessory) may be such that the accessory (crimped closed-cell stent-like accessory, open-cell stent-like accessory, or open-end stent-like accessory) touches the stent catheter and thus has an increased friction (level). In some embodiments, the accessory may be coated with a hydrophilic or a hydrophobic polymeric coating to adjust the friction levels. The coating may have anti-thrombotic properties to reduce coagulation or clotting effects.

As noted above with reference to other embodiments of the catheter accessory 120, one portion of the catheter accessory (crimped closed-cell stent-like accessory, open-cell stent-like accessory, or open-end stent-like accessory) may have a back-end elongated element, which has a constant profile. Fig. 13 illustrates such a back-end elongated element 1300, in accordance with at least one embodiment of the present disclosure. The length of the back-end elongated element 1300 may be such that it extends beyond the length of the guiding catheter so that the operator can manipulate the device by pulling, pushing or torsioning the device while performing cardiovascular interventional procedures. This back-end elongated element may be attached to the accessory (crimped closed-cell stent-like accessory, open-cell stent-like accessory, or open-end stent-like accessory) by means of welding, crimping, gluing, mechanical lock or simply by being part of the same wire element as to form a single-piece device.

In operation, the catheter accessory 120 (crimped closed-cell stent-like accessory, open-cell stent-like accessory, or open-end stent-like accessory) may be mounted on top of the delivery catheter by using a crimping tool (not shown) to collapse the structure in a uniform radial configuration. The crimping tool has the capability of crimping the entire length of the tubular element simultaneously and down to a minimum radius of approximately 0.35 mm. This ensures that the operator may adjust the final crimping diameter and thus control the level of friction of the device against the surface of the intermediate portion of the delivery catheter after installation.

The open-end stent-like structure may have a plurality of interconnecting struts or bridges along its longitudinal axis between the crown-like patterns to modulate the overall flexural stiffness. The length of each circumferential arm may extend in its circumferential length from 50% up to 95% of the of circumference of the tubular structure.

### Hybrid tubular structure (Coil/Braided)

Fig. 14 schematically depicts another embodiment of the catheter accessory 120: a hybrid tubular accessory 1420. The hybrid tubular accessory 1420 has two portions: a coil element 1441 and a braided tubular structure 1442. For example, the coil element 1441 may be made of a metallic or polymeric material. The braided tubular structure 1442 may be made of a metallic or polymeric material. As depicted in Fig. 14, the coil element 1441 and a braided tubular structure 1442 are positioned sequentially along the intermediate portion of the delivery catheter.

The braided tubular structure 1442 may be comprised of a layer of 2 to 32 metallic or polymeric braided filaments embedded in two layers of polymeric material. The combination of these layers forms the braided tubular structure. The braided tubular structure 1442 may also have one or a plurality of backbone elements that may be longitudinally straight or may have a helical shape embedded within the braided structure to reinforce the flexural rigidity of the braided tubular structure 1442.

The overall flexural rigidity of both the coil element and the braided tubular structure 1442 depends on the materials, thickness, and geometrical features. The coil element 1441 may be made of materials such as stainless steel or nitinol. The braided tubular structure 1442 may be made of a combination of stainless-steel wires with polymeric layers that may have low friction properties and the stainless-steel wires may be coated with a hydrophilic or a hydrophobic polymeric coating to adjust the friction levels. The coating may have anti-thrombotic properties to reduce coagulation or clotting effects.

The overall dimensions of the hybrid tubular structure 1442 are such that its internal diameter allows to pass over (cross) the crimped stent outer diameter already mounted on the balloon of the delivery catheter 100. The outer diameter of the hybrid tubular structure 1442 is such that it can fit inside a 5F guiding catheter, or another guiding catheter 110. The overall length of the hybrid tubular structure 1442 is between about 10 cm and about 30 cm, preferably between 15cm and 25 cm, and more preferably about 20cm. The proportional lengths of each segment (coil element or braided tube) may vary and there may be a plurality of those 2 types of segments. In at least one embodiment, the overall combined length of coiled elements and braided elements reaches 100% of the overall length of the hybrid tube.

Mounting of the hybrid tubular structure on top of the stent catheter may be provided by inserting a mounting accessory described above and sliding it across the stent structure of the distal portion of the delivery catheter until it is positioned for its intended clinical use.

To install the catheter accessory 120, the operator slides the hybrid assembly over various devices installed in the distal portion of the catheter, such as a balloon, a stent and/or a crimped valve. In another embodiment, the catheter accessory 120has a lateral 'split' between the braided and coiled elements in order to position the catheter accessory 120 from the proximal part of the delivery catheter. In that preferred embodiment, the 'split' between the braided and coiled elements may be 'horizontal' or 'spiraling' to increase flexibility. The 'split' version might also repeat use during the same procedure.

The outer diameter of each element of the hybrid tubular accessory 1420 (coil element or braided tube) may be equal to or smaller than about 8.0 mm. The inner diameter of each element of the hybrid tubular accessory 1420 (coil element or braided tube) may be equal to or larger than about 0.35 mm. The coil element can be placed at the proximal side of the hybrid structure and thus the braided element may be at the distal side of the hybrid structure or vice-versa. Each element may occupy a length between about 2 cm to 30cm and both braided and coil elements may have an overall length between about 10 cm and about 30 cm in any combination.

### INTERLINKED elements

In at least one embodiment, the catheter accessory 120 is made of interlinked elements along the longitudinal axis of the catheter accessory 120 and each element has different flexural and axial rigidity properties and thus may be adjusted to control the overall stiffness of the catheter 112.

The catheter accessory 120 may be made of a series of thin wires laid longitudinally and assembled inside or attached to a polymeric sheath. The multiple wire arrangement may be circumferential, or intermittent circumferential, or intermittent axial, or shifted arrangements, or may have more wires in one area than another area of the cross-sectional profile of the sheath in order to control the level of stiffness at different portions along the sheath. Moreover, the wires may have different profiles (circular, rectangular or polygonal) to increase or decrease the bending moment of inertia as desired.

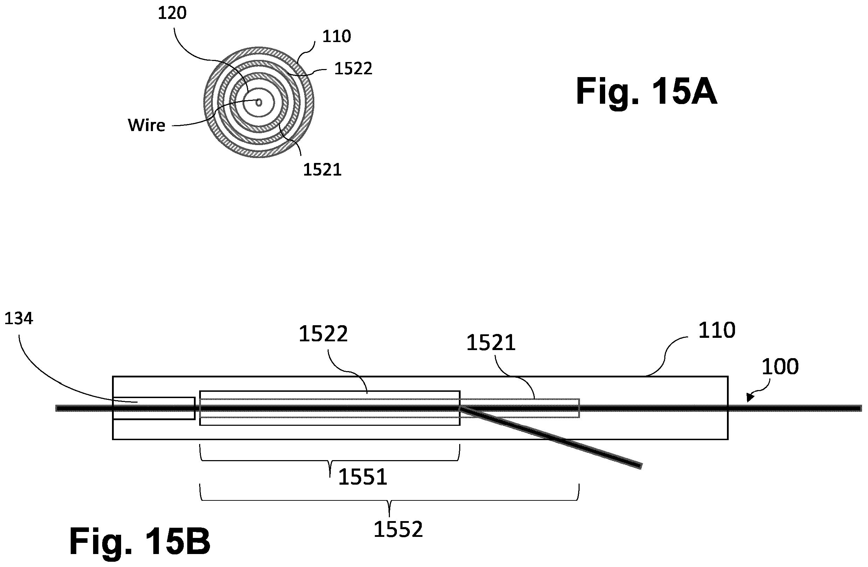

Figs. 15A-15B illustrates another embodiment of the catheter accessory 120: a double-sheath accessory 1520. The double-sheath accessory 1520 has two sheaths: an internal sheath 1521 and an external sheath 1522. The sheaths 1521, 1522 have different lengths 1551, 1552. The length of the external sheath 1522 may be shorter than the length of the internal sheath 1521, as depicted in Fig. 15B. The internal sheath and an external sheath are positioned coaxially with respect to each other and overlapping with respect to a common axis along the intermediate portion of the catheter.

### Hybrid Coil/mesh structure

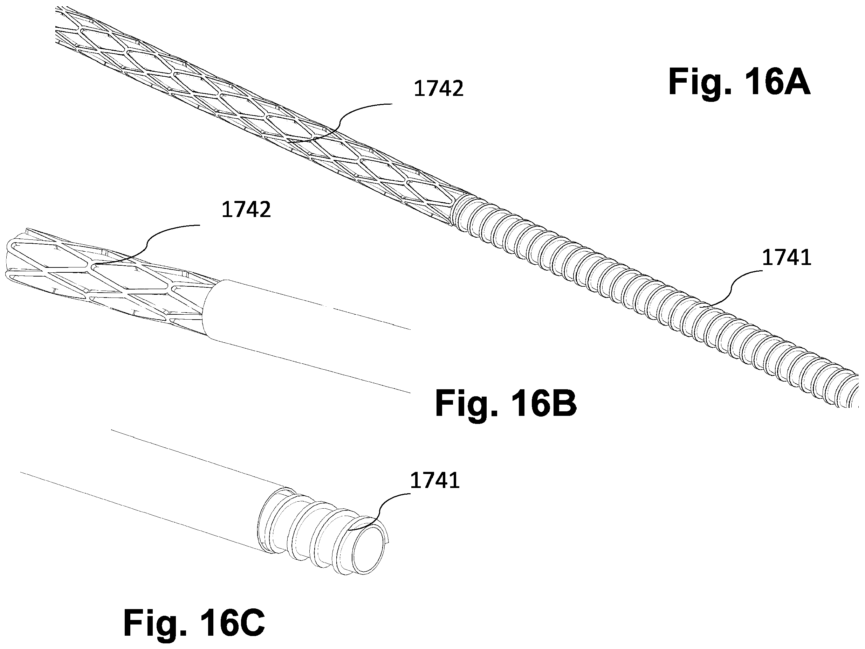

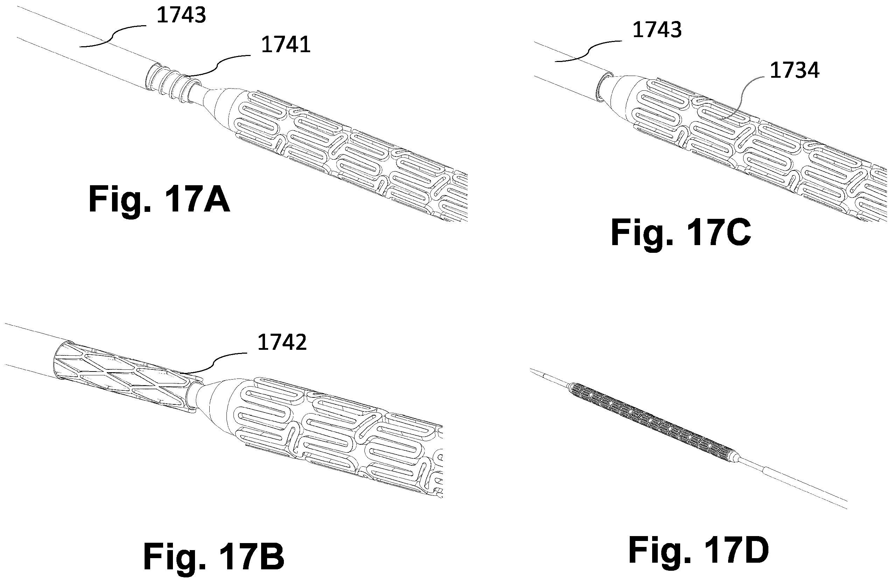

Figs. 16A - 16C illustrate various portions of a coil-mesh hybrid tubular accessory 1720, in accordance with at least one embodiment of the present disclosure. Figs. 17A-17D illustrate coil-mesh hybrid tubular accessory 1720 when installed on the delivery catheter. The coil-mesh hybrid tubular accessory 1720 illustrated in Figs. 16A-17D is a non-limiting example of the hybrid tubular accessory 1420 described above

The coil-mesh hybrid tubular accessory 1720 may be installed with the coiled portion 1741 facing (joining) the distal portion with the stent 1734. Alternatively, the coil-mesh hybrid tubular accessory 1720 may be installed with the stent-like portion 1742 facing (joining) the distal portion with the stent 1734. In some embodiments, the coil-mesh hybrid tubular accessory 1720 may have an additional coating tube 1743 located coaxially with the coiled portion 1741 and the stent-like portion 1742 and covering their outward surface.

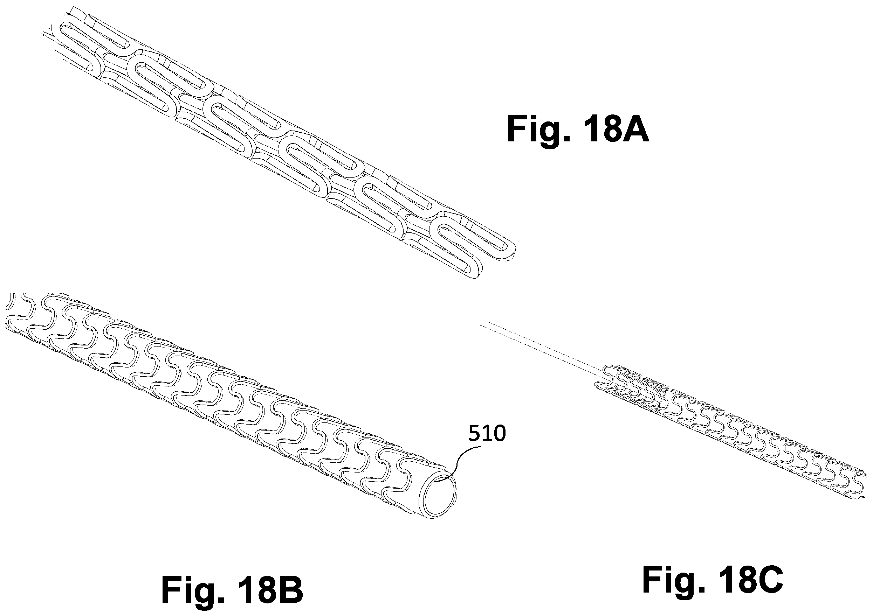

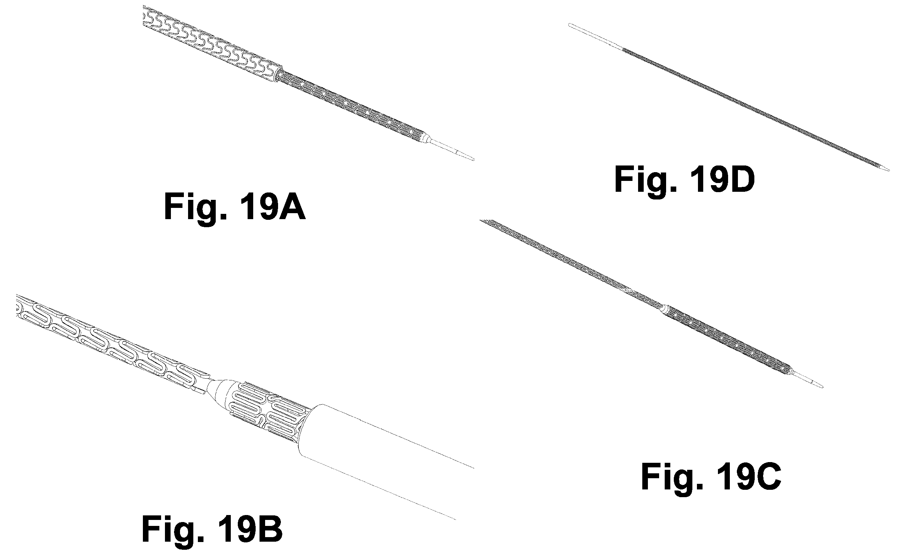

Figs. 18A - 18C illustrate various portions of a wave-like coiled accessory 1820 which is another non-limiting example of the embodiment of the coiled accessory 420 described above. Figs. 19A-19D illustrate the wave-like coiled accessory 1820 when installed on the delivery catheter. The wave-like coiled accessory 1820 is a self-collapsible structure, and the helical path of the coil has a wave-like pattern, that makes it easier for the coil to expand and to self-collapse. The wave-like coiled accessory 1820 depicted in Figs 18A-19D have a sinusoidal pattern of the coil along its helical path which results in improved expansion properties of the wave-like coiled accessory 1820, which simplifies mounting of the wave-like coiled accessory 1820 on a mounting accessory 510 such as a mandrel as described herein. The sinusoidal or wavy pattern along the helical path of the wave-like coiled accessory 1820 allows to control the level of force for radial expansion. The sinusoidal pattern may have three or more waves per revolution of the helical path of the coil.

The catheter accessory as described herein may be used to improve advancing of various medical devices which need to be positioned in a specific anatomical location and which may benefit from the additional support (in terms of supplemental rigidity of the intermediate portion) provided by the catheter accessory. Such medical devices may be, for example, neurovascular interventional devices such as, for example, balloons, stents, flow diverters, aneurysm coils, prosthesis, valves, occluders, and may include vascular as well as non-vascular devices. The catheter accessory as described herein may be also used to improve the pushability of pacemaker leads, for example, in coronary sinus.

The catheter accessory as described herein may provide improvement for any type of neurovascular interventional device where the anatomical features or the pathological lesions represent a challenge to navigate such devices. An example of this are thin devices that crosses through complex or tortuous anatomical features or hard-to-cross lesions. Another example is crossing any neurovascular device through the carotid syphon during neurovascular interventions as the "S" shaped geometry often represents a challenge to cross. The catheter accessory as described herein may be used in any type of peripheral or structural heart interventional procedure: stent-grafts for aneurysm repair, transcatheter aortic valves, mitral clips, Patent Foramen Ovale occluding devices, Patent Ductus arteriosus occluding devices, Amplatzer^{®}-type devices, clot-retrieving applications, pacemaker lead positioning. Other non-cardiovascular applications where the catheter accessory as described herein may be used, are biliary stents, esophageal stents, any type of tubular graft interventional procedure, surgical applications that involve navigating through a cylindrical vessel or small orifices (e.g. endoscopic, laparoscopic or arthroscopic applications).

## Claims

1. A catheter accessory (120) for use with a catheter (112), the catheter (112) having a distal portion (104) comprising an intervention accessory, the catheter (112) having a distal portion outer diameter (335); a proximal portion (106) for handling the catheter (112); and an intermediate portion (108) located between the distal portion (104) and the proximal portion (106) and having an outer intermediate portion diameter (336), the catheter accessory (120) comprising:
an elongated tubular structure configured to coaxially mount on the intermediate portion (108), to attach to the intermediate portion (108) and provide a pre-determined flexural rigidity and a pre-determined flexibility simultaneously to the intermediate portion (108) covered by the elongated tubular structure, the elongated tubular structure having a default shape and size for operation which is **characterized by** a lumen with an inside diameter (325) which is larger than or equal to the intermediate portion outer diameter (336) and smaller than the distal portion outer diameter (335), holding the catheter accessory (112) onto the intermediate portion (108) and completely proximal to the distal portion (104) comprising the intervention accessory during the operation;
**characterized in that**:
the elongated tubular structure is made of a material and shaped to undergo elastic deformation when a force is applied by a mounting accessory from inside outwardly and thereby:
- to expand the catheter accessory (120) radially to a mounting mode by insertion of a mounting accessory (510) into a tubular opening of the catheter accessory (120), the lumen in the mounting mode having an expanded inner diameter (365) larger than the distal portion outer diameter (335) for sliding the distal portion (104) comprising the intervention accessory through and completely across the lumen during installation; and
- to collapse radially back to the default shape and size with an inner operation diameter (325) larger than the outer intermediate portion diameter (336) when the force is not applied anymore from inside outwardly due to removal of the mounting accessory (510) from within the catheter accessory (120), after the distal portion (104) comprising the intervention accessory has slid through and completely across the lumen.

2. The catheter accessory of claim 1, wherein the force is applied along a length of the elongated tubular structure and thereby the elongated tubular structure is configured to expand radially along its length to the mounting mode and to collapse radially back to the default shape and size along the length of the elongated tubular structure.

3. The catheter accessory of any one of claims 1 or 2, wherein the pre-determined flexural rigidity of the elongated tubular structure is between about 0.0001 Pascal x meter⁴ (Pa m⁴) and about 0.002 Pa m⁴.

4. The catheter accessory of any one of claims 1 to 3, wherein the sheath thickness of the elongated tubular structure is between about 0.1 millimeter (mm) and about 3mm.

5. The catheter accessory of any one of claims 1 to 4, wherein the elongated tubular structure is a coiled wire.

6. The catheter accessory of any one of claims 1 to 4, wherein the elongated tubular structure comprises a coil configured to temporarily expand radially by increasing a coil diameter, by an elastic deformation, and to collapse and decrease the coil diameter back an original stress-free diameter.

7. The catheter accessory of any one of claims 1 to 4, wherein the elongated tubular structure has a stent-like structure.

8. The catheter accessory of any one of claims 1 to 4, wherein the elongated tubular structure has circular open-end arms (930) originating from a single backbone element (922).

9. The catheter accessory of any one of claims 1 to 4, wherein the elongated tubular structure is an elastic expandable tubular element (1020) which is capable to radially expand by a deformation without causing a permanent deformation or plasticity and is made of a polymeric material having a high deformability in its elastic zone.

10. The catheter accessory of claim 9, wherein the elastic expandable tubular element (1020) has a draped cross-sectional profile which enhances a radial elasticity of the elastic expandable tubular element (1020).

11. The catheter accessory of claim 9, wherein the elastic expandable tubular element (1020) has a pleated cross-sectional profile with at least one pleat located along the length of the elastic expandable tubular element (1020) which enhances a radial elasticity of the elastic expandable tubular element (1020).

12. The catheter accessory of any one of claims 1 to 4, further comprising a coil element and a braided tubular structure (1442) positioned sequentially along the intermediate portion (108).

13. The catheter accessory of any one of claims 1 to 4, further comprising an internal sheath (1521) and an external sheath (1522) positioned coaxially with respect to each other and overlapping with respect to a common axis along the intermediate portion (108) of the catheter (112).

14. The catheter accessory of claim 1, wherein the catheter (112) is comprised within a guiding catheter (110) having a guiding catheter lumen with an inside guiding catheter diameter, the default shape and size of the elongated tubular structure being further **characterized by** an outside diameter which is smaller than inside guiding catheter diameter, for sliding the catheter accessory (120) from the proximal portion (106) toward the distal portion (104) and be installed for operation proximal to the distal portion (104) comprising the intervention accessory.

15. The catheter accessory of any one of claim 1 to 14, further comprising a back-end elongated element (1300) attached to the elongated tubular structure.

## Patentansprüche

1. Katheterzubehör (120) zur Verwendung mit einem Katheter (112), wobei der Katheter (112) einen distalen Abschnitt (104) aufweist, umfassend ein Interventionszubehör, wobei der Katheter (112) einen distalen Außendurchmesserabschnitt (335) aufweist; einen proximalen Abschnitt (106) zum Handhaben des Katheters (112); und einen Zwischenabschnitt (108), der sich zwischen dem distalen Abschnitt (104) und dem proximalen Abschnitt (106) befindet und einen Außendurchmesserzwischenabschnitt (336) aufweist, das Katheterzubehör (120) umfassend:
eine längliche rohrförmige Struktur, die konfiguriert ist, um koaxial auf dem Zwischenabschnitt (108) montiert zu werden kann, um an dem Zwischenabschnitt (108) angebracht zu werden und dem Zwischenabschnitt (108), der von der länglichen rohrförmigen Struktur bedeckt ist, gleichzeitig eine vorbestimmte Biegesteifigkeit und eine vorbestimmte Flexibilität zu verleihen, wobei die längliche rohrförmige Struktur eine Standardform und - größe für einen Betrieb aufweist, die durch ein Lumen mit einem Innendurchmesser (325) gekennzeichnet ist, der größer als oder gleich wie der Außendurchmesserzwischenabschnitt (336) und kleiner als der distale Außendurchmesserabschnitt (335) ist, Halten des Katheterzubehörs (112) auf dem Zwischenabschnitt (108) und vollständig proximal zu dem distalen Abschnitt (104), umfassend das Interventionszubehör während der Operation;
**dadurch gekennzeichnet, dass**:
die längliche, rohrförmige Struktur ist aus einem Material gefertigt und geformt ist, um eine elastische Verformung zu erfahren, wenn eine Kraft durch ein Montagezubehör von innen nach außen angewandt wird und dadurch:
- um das Katheterzubehör (120) durch Einsetzen eines Montagezubehörs (510) in eine rohrförmige Öffnung des Katheterzubehörs (120) radial in einen Montagemodus zu erweitern, wobei das Lumen in dem Montagemodus einen erweiterten Innendurchmesser (365) aufweist, der größer ist als der distale Außendurchmesserabschnitt (335), um den distalen Abschnitt (104), der das Interventionszubehör umfasst, während der Installation durch das Lumen hindurch und vollständig darüber zu schieben; und
- radial wieder auf die Standardform und -größe mit einem inneren Operationsdurchmesser (325), der größer ist als der äußere Zwischenabschnittsdurchmesser (336), zu kollabieren, wenn die Kraft nicht mehr von innen nach außen ausgeübt wird, weil das Montagezubehör (510) aus dem Katheterzubehör (120) entfernt wurde, nachdem der distale Abschnitt (104), der das Interventionszubehör umfasst, durch das Lumen vollständig geschoben wurde.

2. Katheterzubehör nach Anspruch 1, wobei die Kraft entlang einer Länge der länglichen rohrförmigen Struktur angewandt wird und dadurch die längliche rohrförmige Struktur konfiguriert ist, um sich radial entlang ihrer Länge bis zu dem Montagemodus auszudehnen und radial zurück auf die Standardform und -größe entlang der Länge zusammenzufallen.

3. Das Katheterzubehör nach einem der Ansprüche 1 oder 2, wobei die vorbestimmte Biegesteifigkeit der länglichen rohrförmigen Struktur zwischen etwa 0,0001 Pascal × Meter⁴ (Pa m⁴) und etwa 0,002 Pa m⁴ ist.

4. Katheterzubehör nach einem der Ansprüche 1 bis 3, wobei die Mantelstärke der länglichen rohrförmigen Struktur zwischen etwa 0,1 Millimeter (mm) und etwa 3mm ist.

5. Katheterzubehör nach einem der Ansprüche 1 bis 4, wobei die längliche rohrförmige Struktur ein gewickelter Draht ist.

6. Katheterzubehör nach einem der Ansprüche 1 bis 4, wobei die längliche rohrförmige Struktur eine Spule umfasst, die konfiguriert ist, um sich durch elastische Verformung vorübergehend radial auszudehnen, indem sie einen Spulendurchmesser vergrößert, und um zu kollabieren und den Spulendurchmesser wieder auf einen ursprünglichen spannungsfreien Durchmesser zu verringern.

7. Katheterzubehör nach einem der Ansprüche 1 bis 4, wobei die längliche rohrförmige Struktur eine stentartige Struktur aufweist.

8. Katheterzubehör nach einem der Ansprüche 1 bis 4, wobei die längliche rohrförmige Struktur kreisförmige Arme (930) mit offenen Enden aufweist, die von einem einzigen Rückgrat (922) ausgehen.

9. Katheterzubehör nach einem der Ansprüche 1 bis 4, wobei die längliche rohrförmige Struktur ein elastisch ausdehnbares rohrförmiges Element (1020) ist, das in der Lage ist, sich durch Verformung radial auszudehnen, ohne eine dauerhafte Verformung oder Plastizität zu verursachen, und das aus einem polymeren Material gefertigt ist, das eine hohe Verformbarkeit in seiner elastischen Zone aufweist.

10. Katheterzubehör nach Anspruch 9, wobei das elastisch ausdehnbare rohrförmige Element (1020) ein drapiertes Querschnittsprofil aufweist, das die radiale Elastizität dieses Elements (1020) erhöht.

11. Katheterzubehör nach Anspruch 9, wobei das elastisch ausdehnbare rohrförmige Element (1020) ein gefaltetes Querschnittsprofil mit mindestens einer Falte aufweist, die entlang der Länge des elastisch ausdehnbaren rohrförmigen Elements (1020) angeordnet ist, was die radiale Elastizität des elastisch ausdehnbaren rohrförmigen Elements (1020) erhöht.

12. Katheterzubehör nach einem der Ansprüche 1 bis 4, ferner umfassend ein Spulenelement und eine geflochtene rohrförmige Struktur (1442), die nacheinander entlang des Zwischenabschnitts (108) positioniert sind.

13. Katheterzubehör nach einem der Ansprüche 1 bis 4, ferner umfassend eine innere Hülle (1521) und eine äußere Hülle (1522), die koaxial zueinander positioniert sind und sich in Bezug auf eine gemeinsame Achse entlang des Zwischenabschnitts (108) des Katheters (112) überlappen.

14. Katheterzubehör nach Anspruch 1, wobei der Katheter (112) in einem Führungskatheter (110) enthalten ist, der ein Lumen mit einem inneren Führungskatheterdurchmesser aufweist, und wobei die Standardform und -größe der länglichen rohrförmigen Struktur ferner durch einen Außendurchmesser gekennzeichnet ist, der kleiner ist als der innere Führungskatheterdurchmesser, um das Katheterzubehör (120) von dem proximalen Abschnitt (106) zu dem distalen Abschnitt (104) zu schieben und proximal zu dem distalen Abschnitt (104), der das Interventionszubehör umfasst, für den Betrieb installiert zu werden.

15. Katheterzubehör nach einem der Ansprüche 1 bis 14, ferner umfassend ein längliches Element (1300) an dem hinteren Ende, das an der länglichen rohrförmigen Struktur angebracht ist.

## Revendications

1. Accessoire de cathéter (120) à utiliser avec un cathéter (112), le cathéter (112) ayant une partie distale (104) comprenant un accessoire d'intervention, le cathéter (112) ayant un diamètre extérieur de partie distale (335) ; une partie proximale (106) pour manipuler le cathéter (112) ; et une partie intermédiaire (108) située entre la partie distale (104) et la partie proximale (106) et ayant un diamètre extérieur de partie intermédiaire (336), l'accessoire de cathéter (120) comprenant :
une structure tubulaire allongée configurée pour se monter coaxialement sur la partie intermédiaire (108), pour se fixer à la partie intermédiaire (108) et fournir simultanément une rigidité en flexion prédéterminée et une flexibilité prédéterminée à la partie intermédiaire (108) couverte par la structure tubulaire allongée, la structure tubulaire allongée présentant, pour fonctionner, une forme et une taille par défaut **caractérisées par** une lumière ayant un diamètre intérieur (325) qui est supérieur ou égal au diamètre extérieur de partie intermédiaire (336) et inférieur au diamètre extérieur de partie distale (335), maintenant l'accessoire de cathéter (112) sur la partie intermédiaire (108) et entièrement proximal à la partie distale (104) comprenant l'accessoire d'intervention au cours de l'utilisation ;
**caractérisé en ce que** :
la structure tubulaire allongée est constituée d'un matériau et conformée pour subir une déformation élastique lorsqu'une force est appliquée par un accessoire de montage de l'intérieur vers l'extérieur et ainsi :
- déployer radialement l'accessoire de cathéter (120) en mode de montage par l'insertion d'un accessoire de montage (510) dans une ouverture tubulaire de l'accessoire de cathéter (120), la lumière en mode montage ayant un diamètre intérieur étendu (365) supérieur au diamètre extérieur de partie distale (335) pour faire coulisser la partie distale (104) comprenant l'accessoire d'intervention à travers et sur toute la longueur de la lumière lors de l'installation ; et
- se rétracter radialement pour retrouver la forme et la taille par défaut avec un diamètre de fonctionnement intérieur (325) supérieur au diamètre extérieur de partie intermédiaire (336) lorsque la force n'est plus appliquée de l'intérieur vers l'extérieur en raison du retrait de l'accessoire de montage (510) de l'intérieur de l'accessoire de cathéter (120), après que la partie distale (104) comprenant l'accessoire d'intervention a coulissé complètement à travers la lumière.

2. Accessoire de cathéter selon la revendication 1, dans lequel la force est appliquée sur une longueur de la structure tubulaire allongée et la structure tubulaire allongée est ainsi configurée pour s'étendre radialement sur sa longueur jusqu'au mode de montage et pour se rétracter radialement jusqu'à la forme et la taille par défaut sur la longueur de la structure tubulaire allongée.

3. Accessoire de cathéter selon l'une quelconque des revendications 1 ou 2, dans lequel la rigidité en flexion prédéterminée de la structure tubulaire allongée est comprise entre environ 0,0001 Pascal x mètre⁴ (Pa m⁴) et environ 0,002 Pa m⁴.

4. Accessoire de cathéter selon l'une quelconque des revendications 1 à 3, dans lequel l'épaisseur de la gaine de la structure tubulaire allongée est comprise entre environ 0,1 millimètre (mm) et environ 3 mm.

5. Accessoire de cathéter selon l'une quelconque des revendications 1 à 4, dans lequel la structure tubulaire allongée est un fil enroulé.

6. Accessoire de cathéter selon l'une quelconque des revendications 1 à 4, dans lequel la structure tubulaire allongée comprend une bobine configurée pour s'étendre temporairement radialement en augmentant un diamètre de bobine, par une déformation élastique, et pour se rétracter et diminuer le diamètre de la bobine afin de revenir à un diamètre original sans contrainte.

7. Accessoire de cathéter selon l'une quelconque des revendications 1 à 4, dans lequel la structure tubulaire allongée est de type endoprothèse.

8. Accessoire de cathéter selon l'une quelconque des revendications 1 à 4, dans lequel la structure tubulaire allongée comporte des bras circulaires à extrémité ouverte (930) partant d'un élément d'ossature unique (922).

9. Accessoire de cathéter selon l'une quelconque des revendications 1 à 4, dans lequel la structure tubulaire allongée est un élément tubulaire extensible élastique (1020) qui est capable de s'étendre radialement par déformation sans provoquer de déformation permanente ou de plasticité et est constitué d'un matériau polymère ayant une grande capacité de déformation dans sa zone d'élasticité.

10. Accessoire de cathéter selon la revendication 9, dans lequel l'élément tubulaire extensible élastique (1020) a un profil de section transversale drapé qui améliore l'élasticité radiale de l'élément tubulaire extensible élastique (1020).

11. Accessoire de cathéter selon la revendication 9, dans lequel l'élément tubulaire extensible élastique (1020) présente un profil de section transversale plissé avec au moins un pli situé sur la longueur de l'élément tubulaire extensible élastique (1020), ce qui améliore une élasticité radiale de l'élément tubulaire extensible élastique (1020).

12. Accessoire de cathéter selon l'une quelconque des revendications 1 à 4, comprenant en outre un élément de bobine et une structure tubulaire tressée (1442) positionnée successivement le long de la partie intermédiaire (108).

13. Accessoire de cathéter selon l'une quelconque des revendications 1 à 4, comprenant en outre une gaine intérieure (1521) et une gaine extérieure (1522) positionnées coaxialement l'une par rapport à l'autre et se chevauchant par rapport à un axe commun le long de la partie intermédiaire (108) du cathéter (112).

14. Accessoire de cathéter selon la revendication 1, dans lequel le cathéter (112) est contenu dans un cathéter de guidage (110) comportant une lumière de cathéter de guidage ayant un diamètre intérieur de cathéter de guidage, la forme et la taille par défaut de la structure tubulaire allongée étant en outre **caractérisées par** un diamètre extérieur qui est inférieur au diamètre intérieur de cathéter de guidage, pour faire coulisser l'accessoire de cathéter (120) de la partie proximale (106) vers la partie distale (104) et l'installer pour son utilisation de manière proximale à la partie distale (104) comprenant l'accessoire d'intervention.

15. Accessoire de cathéter selon l'une quelconque des revendications 1 à 14, comprenant en outre un élément arrière allongé (1300) fixé à la structure tubulaire allongée.
